# EUROPEAN PATENT SPECIFICATION

(11) **EP 1 353 699 B1**
(45) Date of publication and mention of the grant of the patent: **01.02.2012**
(21) Application number: 01271230.3
(22) Date of filing: 20.12.2001
(51) Int. Cl.: A61K 47/48, A61K 31/585, B82Y 5/00, A61P 15/18, A61P 15/12

(54) **Beta-cyclodextrin-drospirenone inclusion complexes**
Einschlusskomplexe von Drospirenone und Beta-Cyclodextrin
Complexes d'inclusion béta-cyclodextrine-drospirenone

(30) Priority: 20.12.2000 EP 00610134; 20.12.2000 US 256483 P
(43) Date of publication of application: 22.10.2003
(73) Proprietor: Bayer Pharma Aktiengesellschaft, 13353 Berlin (DE)
(72) Inventor: BACKENSFELD, Thomas, 13127 Berlin (DE); HEIL, Wolfgang, 21435 Stelle (DE)
(74) Representative: Plougmann & Vingtoft A/S
(86) International application number: PCT/IB2001/002604
(87) International publication number: WO 2002/049674

(56) References cited:
- EP-A- 0 398 460
- WO-A-96/02277
- WO-A1-01/15701
- FR-A- 2 515 187
- GB-A- 2 109 381
- KRATTENMACHER ROLF: "Drospirenone: Pharmacology and pharmacokinetics of a unique progestogen." CONTRACEPTION, vol. 62, no. 1, July 2000 (2000-07), pages 29-38, XP000993492 ISSN: 0010-7824
- NORMAN P ET AL: "Drospirenone: Contraceptive, hormone replacement therapy, aldosterone antagonist, progestogen: 1,2-dihydrospirorenone, SH-470, ZK-30595." DRUGS OF THE FUTURE, vol. 25, no. 12, December 2000 (2000-12), pages 1247-1256, XP000993474 ISSN: 0377-8282
- UEKAMA, K. ET AL: "Inclusion complexations of steroid hormones with cyclodextrins in water and in solid phase" INT. J. PHARM., 1982, VOL. 10, NO. 1, PAGES 1-15, XP000610489
- KRALOVA, K. ET AL: "Interactions of beta -cyclodextrin with steroid compounds in aqueous solutions" PHARMAZIE, 1989, VOL. 44, NO. 9, PAGES 623-5, XP000611476
- HERMENS W.A.J.J.: "Delivery of hormones: Some new concepts" PHARM. WEEKBL. SCI. ED., 1992, VOL. 14, NO. 4 A, PAGE(S) 253-257, XP002167344 NETHERLANDS
- K. UEKAMA: "CYCLODEXTRIN INCLUSION COMPOUNDS: EFFECTS ON STABILITY AND BIO-PHARMACEUTICAL PROPERTIES" TOPICS IN PHARMACEUTICAL SCIENCES, EDS. D.D. BREIMER AND P. SPEISER, 1987, ELSEVIER SCIENCE PUBLISHERS B.V. (BIOMEDICAL DIVISION), PAGES 181-194, XP002167345
- UEKAMA K ET AL: "CYCLODEXTRIN DRUG CARRIER SYSTEMS" CHEMICAL REVIEWS,US,AMERICAN CHEMICAL SOCIETY. EASTON, vol. 98, no. 5, 1 July 1998 (1998-07-01), pages 2045-2076, XP000771829 ISSN: 0009-2665
- DALLA BELLA M ET AL: "CYCLODEXTRINS", DRUGS OF THE FUTURE, PROUS SCIENCE, ES, vol. 8, no. 5, 1 January 1983 (1983-01-01) , pages 391-394, XP008020709, ISSN: 0377-8282
- MELIA C D ET AL: "Review article: Mechanisms of drug release from tablets and capsules. 2. Dissolution", ALIMENTARY PHARMACOLOGY AND THERAPEUTICS, vol. 3, no. 6, 1989, pages 513-525, ISSN: 0269-2813

## Description

### FIELD OF INVENTION

The present invention relates to an inclusion complex formed between β-cyclodextrin and drospirenone and to methods of providing such an inclusion complex. Moreover, the present invention relates to the use of said inclusion complex in pharmaceutical compositions for use as a medicament in the treatment of symptoms associated with menopause and in female contraception.

### BACKGROUND OF THE INVENTION

Drospirenone (6β,7β;15β,16β-dimethylene-3-oxo-17α-pregn-4-ene-21,17-carbolactone), which may be prepared substantially as described in e.g. US 4,129,564 or WO 98/06738, is only sparingly soluble in aqueous media at various pH values.

The water solubility of a compound is extremely pertinent with regards to its utility In industry, particularly in the pharmaceutical industry where there is a strong link between water solubility and bioavailability. The therapeutic efficiency of drospirenone may be improved by increasing its overall water solubility, thus providing for routes of administration alternative to those proceeding via the gastrointestinal tract, where absorption is slow and then rapidly cleared from circulating blood by the liver.

Cyclodextrins are known to solubilize nonpolar compounds and improve the absorption of certain compounds by forming complexes with said compounds. The cyclodextrins are frequently derivatized in order to improve the solubility or to accommodate appropriately the compound of interest. However, certain compounds are not well accommodated by the cavity of the some of the cyclodextrin molecules.

Drospirenone, in its uncomplexed form, is known from DE 26 52 761 in which its use as a diuretic compound is disclosed.

EP 0 398 460 described the use of drospirenone for contraception and for symptoms associated with menopause.

U5 4,596,795 discloses a complex between α-, p- and γ-cyclodextrins and derivatives thereof with testosterone, progesterone, and estradiol and the solubility of said complexes.

US 5,885,978 relates to a composition comprising an adrenal cortical steroid and cyclodextrin prepared by clathrating the adrenal cortical steroid in the cyclodextrin using a homomixer.

US 5,376,641 discloses a method of making a steroid water soluble by mixing a steroid and a branched beta cyclodextrin together in water for a period of 4 to 24 hours under ambient conditions.

US 5,376,641 discloses a method for making a steroid water soluble by complexing the steroid with branched β-cyclodextrin.

US 4,727,064 discloses a method of improving the dissolution properties of.a steroid by forming a solid comprising at least one of testosterone, progesterone and estradiol as an inclusion complex with a poly-β-cyclodextrin and /or hydroxypropyl-β-cyclodextrin adapted for administration by buccal route.

FR 2 515 187 discloses inclusion complexes between γ-cyclodextrines and various steroids, such as a spironolactone steroid.

WO 96/02277 discloses pharmaceutical compositions containing cyclodextrin-clathrate complexes of steroid sexual hormones for protection against oxidative degradation of steroids.

### SUMMARY OF THE INVENTION

The invention relates to an inclusion complex between β-cyclodextrin and 6β,7β; 15β,16β-dimethylene-3-oxo-17α-pregn-4-ene-21,17-carbolactone (drospirenone) where the molar ratio between drospirenone and the β-cyclodextrin is 1:3.

The invention also relates to a method for producing the inclusion complex according to the invention.

One object of the present invention is to increase the water-solubility of drospirenone. The present invention thus further discloses methods for improving the solubility of drospirenone, said method comprising forming an inclusion complex between drospirenone and β-cyclodextrin.

In a further aspect of the invention, pharmaceutical compositions comprising an inclusion complex of drospirenone and β-cyclodextrin are described. Consequently, the inclusion complex between drospirenone and β-cyclodextrin for use for female contraception or for the treatment of menopausal symptoms are defined herein.

### DETAILED DESCIPTION OF THE INVENTION

The term "inclusion complex" is intended to mean a complex wherein at least a moiety of drospirenone has inserted itself, at least partially, into the cavity of cyclodextrin.

In efforts to improve the functional utility of drospirenone, research has led-to a new chemical entity, an inclusion complex between β- cyclodextrin and drospirenone in a motar ratio of 3:1.

The β-cyclodextrin may be modified such that some or all of the primary or secondary hydroxyls of the macrocyle, or both, may be alkylated or acylated. Methods of modifying these alcohols are well known to the person skilled in the art and many derivatives are commercially available. The β-cyclodextrin may be modified such that one or more of the primary or secondary hydroxyls of the macrocyle, or both, may be alkylated or acylated. Methods of modifying these alcohols are well known to the person skilled in the art and many are commercially available. Thus, some or all of the hydroxyls of β-cyclodextrin may have be substituted with an O-R group or an O-C(O)-R, wherein R is an optionally substituted C₁₋₆ alkyl, an optionally substituted C₂₋₆ alkenyl, an optionally substituted C₂₋₆ alkynyl, an optionally substituted aryl or heteroaryl group. R may be methyl, ethyl, propyl, butyl, pentyl, or hexyl group. Consequently, O-C(O)-R may be an acetate. Furthermore, R may be such as to derivatize β-cyclodextrin with the commonly employed 2-hydroxyethyl group, or 2-hydroxypropyl group. Moreover, the β-cyclodextrin alcohols may be perbenzylated, per-benzoylated, or benzylated or benzoylated on just one face of the macrocycle, or wherein only 1, 2, 3, 4, 5, or 6 hydroxyls are benzylated or benzoylated. The hydroxyl groups of β-cyclodextrin may be per-alkylated or per-acylated such as permethylated or per-acetylated, or alkylated or acylated, such as methylated or acetylated, on just one face of the macrocycle, or wherein only 1, 2, 3, 4, 5, or 6 hydroxyls are alkylated or acylated, such as methylated or acetylated.

Without being limited to a particular manner in which the inclusion complex is formed, it is presumed that the inclusion complex is an inclusion complex wherein hydrophobic interactions favour the inclusion of hydrophobic moieties from drospirenone into the cavity of a β-cyclodextrin molecule, given the relative hydrophobicity of the numerous alkyl groups in the cavity of the β-cyclodextrin.

The term "solubility" In connection with drospirenone is intended to mean the solubility of the inclusion complex between drospirenone and β-cyclodextrin in water.
The term "total solubility" relates to the drospirenone concentration in a phase solubility isotherm, namely to the solubility of uncomplexed and complexed drospirenone. The "total solubility" is a function of the β-cyclodextrin concentration.

Given one of the objects of the present invention is to increase the solubility and total solubility of drospirenone, it is preferred that the inclusion complex is such that the total water solubility of drospirenone at 20°C is increased by a factor of at least 2, such as at least 2.5, at least 3, at least 3.5, or at least 4 compared to drospirenone in an uncomplexed form.

Correspondingly, it is preferred that the total solubility of drospirenone in water at 20°C is increased to at least 9 x 10⁻⁵ mol/L, such as at least 1 x 10⁻⁴ mol/L, 2 x 10⁻⁴ mol/L, 3 x 10⁻⁴ mol/L or 3.5 x 10⁻⁴ mol/L.

The inclusion complex may exist in the form of a hydrate containing varying amounts of water, such as between about 1% and 25% water. The degree of hydration may vary according to, amongst other reasons, the degree of substitution of the hydroxyls and the method of preparation. The water content of the inclusion complex may depend on the manner in which the inclusion complex is stored, the temperature, pressure and relative humidity. Thus, any discussion on the solid state form of the drospirenone-cyclodextrin inclusion complex comprises the range of hydrates. The hydrate water is part of the crystal lattice and thus modifying the water content may change the crystal lattice and possibly some of the physical properties of the inclusion complex.

As is known to the person skilled in the art, β-cyclodextrin itself forms an inclusion complex with water. Thus, the β-clodextrin used in the preparation of the drospirenone-β-cyclodextrin inclusion complex may be in a hydrated form or in an anhydrous form.

A further object of the invention is to provide a method for producing an inclusion complex comprising the step of combining β-cylodextrin and drospirenone at a molar ratio of 0.3:1 to 20:1, preferably 1:1, 2:1, 3:1, 4:1 or 5:1, most preferably 2:1 or 3:1, particularly 3:1.

The term "solution" in connection with β-cyclodextrin or drospirenone and in connection with the preparation of an inclusion complex is intended to comprise embodiments wherein the solute, namely β-cyclodextrin or drospirenone, is fully or partially dissolved in the solvent so as to form a homogenous solution, a saturated solution, a super-saturated solution, a slurry or a suspension.

In the preparation of the inclusion complex according to the present invention, the combining of the components may be done using a solution of β-cyclodextrin, comprising organic solvent or an aqueous solution such as water. In sensible embodiments of the invention, the solvent comprises a mixture of water and an organic solvent. The organic solvent may be selected from any of those commonly used in organic synthesis such as, but not limited to, THF, methylene chloride, diethyl ether, petroleum ether, ethyl acetate, dioxane, DMF, DMSO, acetone, acetonitrile, ethanol, methanol, pyridine, or combinations thereof. Preferably, the organic solvent is miscible with water. Polar solvents are preferred such as water, methanol, ethanol, DMSO, DMF, and pyridine, most preferably water or ethanol, particularly water.

A solution of β-cyclodextrin, as described *supra,* in any concentration or degree of homogeneity, may be combined with solid drospirenone. Alternatively, the β-cyclodextrin solution may be combined with a solution of drospirenone. In the embodiment where a solution of β-cyclodextrin is combined with solid drospirenone, drospirenone may be In its micronized form.

In the embodiment where a solution of β-cyclodextrin is combined with a solution of drospirenone, drospirenone may be fully or partly dissolved in an organic solvent or water. Organic solvents may be selected from any of those known to the person skilled in the art such as, but not limited to, THF; methylene chloride, diethyl ether, petroleum ether, ethyl acetate, dioxane, DMF, DMSO, acetone, acetonitrile, ethanol, methanol, pyridine, or combinations thereof.

It follows that a solution of drospirenone, as *described supra,* in any degree of homogeneity and in any concentration may be combined with solid β-cyclodextrin in the preparation of an inclusion complex between β-cyclodextrin and drospirenone.

Alternatively, solid drospirenone and solid β-cyclodextrin may be combined in their solid forms and then combined with water or an organic solvent.

In a preferred embodiment of the invention, a method of producing an inclusion complex comprises the steps of dissolving β-cyclodextrin in water, optionally with the aid of heating, to form a β-cyclodextrin solution; dissolving drospirenone in a solvent selected from the group comprising of water and ethanol or mixtures thereof, optionally with the aid of heating, to form a drospirenone solution; combining the β-cyclodextrin solution and the drospirenone solution to form a combined solution; stirring the combined solution, preferably while keeping the solution at or below 25°C; filtering the resultant precipitate; washing the precipitate with a solvent selected from the group consisting of water, ethanol, ether and acetone, preferably wherein the solvent is cooled to below 25°C; optionally suspending the resultant solid In a solvent, preferably acetone, and washing the suspended material with a solvent selected from the group consisting of water, ethanol, ether and acetone, preferably wherein the solvent is cooled to below 25°C; removing substantially a of the solvent from the solid material. Preferably, the solvent is removed by spray drying or alternatively by lyophilization.

The method of preparation may further comprise mechanical mixing, agitation or shaking, or heating of the solutions or combined components.

In embodiment of the invention wherein an organic-solvent is used in the combination of drospirenone or β-cyclodextrin; the inclusion complex formed may contain one or more molecules of said solvents, depending on the method of drying, precipitation or crystallisation. The complex may alternatively exist in the form of a hydrate containing varying amounts of water.

A typical preparation of the drospirenone-β-cyclodextrin inclusion complex may be as follows: Drospirenone is dissolved in a solvent such as acetone or ethanol. The β-cyclodextrin is dissolved in water between 20 and 100°C, such as between 30 and 90°C, such as between 40 and 80°C, preferably between 40 and 60°C, such as at or near 40°C, 45°C, 50°C, 55°C or 60°C. The drospirenone solution is added to the β-cyclodextrin solution and the obtained suspension is stirred at 20-30°C for some hours, such as about 0.5 to 48 hours, then stirred at 2°C for some hours. The crystallised product is isolated and dried. In an alternative process, the drospirenone solution is added to the β-cyclodextrin solution and the obtained suspension is stirred at temperatures below 25°C.

The inclusion complex may be prepared by methods described in or similar to those described in Examples 2 and 3.

The crystallised product may be washed with water, acetone and/or any other solvent in order to wash off non-complexed material. The solvent used to wash the crystallised product may be pre-cooled to below 25°C. This crystallised product may be dried over a drying agent such as P₂O₅ or any other known to the person skilled in the art in a vacuum dessicator or cabinet for several hours or days. It may also be cooled in the dessicator during drying, or undergo spray drying or lyophillization.

A further objective of the invention is to provide a pharmaceutical composition comprising an inclusion complex of drospirenone and cyclodextrin as described *supra* together with one or more pharmaceutically acceptable carriers or excipients. The pharmaceutical composition may be adapted to be administered by oral, parental, mucosal, or topical, vaginal, subcutaneous or nasal administration. The composition may comprise from 0.1 mg to 10 mg of drospirenone, depending on its therapeutic application.

The drospirenone β-cyclodextrin inclusion complex may be used as a medicament. The drospirenone β-cyclodextrin inclusion complex may be used for the preparation of a pharmaceutical composition for female contraception or for the treatment of menopausal symptoms.

In suitable embodiments of the present invention, a pharmaceutical composition may comprise of an inclusion complex between drospirenone and β-cyclodextrin and further comprise of one or more therapeutically active substances. The therapeutically active substance is preferable a steroid. The therapeutically active substance may be complexed with cyclodextrin. The pharmaceutical composition may comprise of a drospirenone β-cyclodextrin inclusion complex, a therapeutically active substance such as estrogen or progestogen or a gestagen together with one or more pharmaceutically acceptable carriers or exciplents.

### BRIEF DESCRIPTION OF THE EXAMPLES

Example 1 compares the solubility of drospirenone in water with the solubility of a sample of an inclusion complex substantially consisting of a 1:1 molar ratio between β-cyclodextrin and drospirenone and to a sample consisting substantially of a 2:1 molar ratio between β-cyclodextrin and drospirenone. The example illustrates the increase in solubility of drospirenone by complexation with β-cyclodextrin. The example further discloses the stability of the 1:1 complex.

Examples 2 and 3 disclose two alternative methods for the preparation of a complex between drospirenone and β-CD.

### BRIEF DESCRIPTION OF THE FIGURES

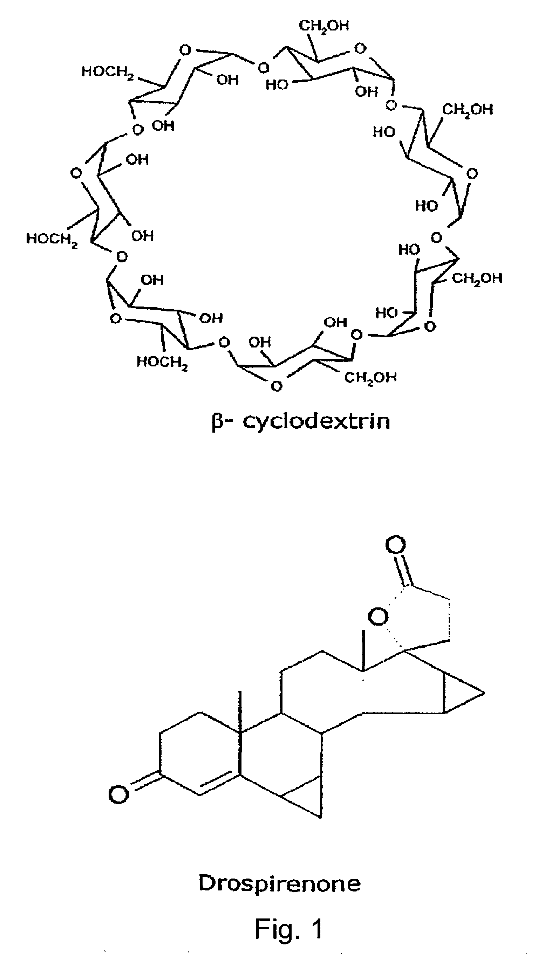

Figure 1 represents the structure of drospirenone β-cyclodextrin. β-cyclodextrin is a macrocycle consisting of 7 sugar units.

### EXAMPLES

### Example 1

### Solubility of drospirenone

The following data were obtained with the phase solubility diagram technique (PSD) In aqueous solutions at 20°C. The stability constants of the inclusion compound from β-CD and drospirenone are given.

| | |
|---|---|
| Stability constant of the 1:1 complex | K₁₁ = 2.2 x 10⁻⁴ M⁻¹ |
| Solubility of Drospirenone | S_{DP} = 4.14 x 10⁻⁵ mol/L (1.51 X 10⁻² g/L) |
| Solubility of 1:1 complex | S_{1:1} = 3.88 x 10⁻⁴ mol/L (0.516 g/L) |
| Solubility of 1:2 complex | S_{1:2} = 3.79 x 10⁻⁵ mol/L (0.1 g/L) |

### Example 2

### Preparation of a complex between drospirenone and β-CD

24 mmol of the β-cyclodextrin are dissolved in 970 mL of water at 45°C and, over the course of 30 min, 8 mmol of drospirenone, dissolved In 90 mL of ethanol are added dropwise. After washing with a further 5 mL of ethanol, cooling to room temperature, stirring at room temperature for 22 h and stirring in an ice bath (4°C) for 3 h, the precipitate was filtered off with suction on a G4 frit. The resulting complex was then washed twice with 100 mL of ice water each time and twice with 50 mL of ice-cooled acetone. It is then dried in a dessicator over phosphorous pentoxide.

### Example 3

### Preparation of a complex between drospirenone and β-CD

15.5 g of β-CD are dissolved in 1000 mL of water, heating if necessary. 1.468 g of drospirenone are weighed into the aqueous cyclodextrin solution. The suspension is stirred at room temperature for 72 h. It is then stirred at +2°C for 3 h. The solid is filtered off with suction on a G4 frit and washed twice with 100 mL of water each time. The crystals are twice suspended in 50 mL of acetone and filtered off with suction each time. They are then washed with 100 mL of water. The moist crystals are dried *in vacuo* over phosphorous pentoxide.

## Claims

1. An inclusion complex between β-cyclodextrin and drospirenone, wherein the inclusion complex between drospirenone and the β-cyclodextrin is in molar ratio of 1:3.

2. The inclusion complex according to claim 1, further comprising one or more therapeutically active substances.

3. A method for producing an inclusion complex as defined in claim 1 comprising combining β-cyclodextrin and drospirenone at a molar ratio of 0.3:1 1 to 20: 1.

4. The method according to claim 3, comprising the combining of a solution of drospirenone with a solution of β-cyclodextrin.

5. The method according to claim 3, comprising the combining of solid drospirenone with a solution of β-cyclodextrin.

6. The method according to claim 4 or 5, wherein the solution of β-cyclodextrin comprises a solvent selected from the group consisting of water, ethanol, acetone, acetonitrile, methanol, DMSO, pyridine or combinations thereof, preferably water.

7. The method according to claim 4, wherein the solution of drospirenone comprises a solvent selected from the group consisting of water, ethanol, acetone, acetonitrile, methanol, DMSO, pyridine or combinations thereof, preferably water or ethanol.

8. The method according to claim 3 comprising the steps of;
dissolving β-cyclodextrin in water, optionally with heating, to form a β-cyclodextrin solution;
dissolving drospirenone in a solvent selected from the group consisting of water and ethanol or mixtures thereof, optionally with heating, to form a drospirenone solution;
combining the β-cyclodextrin solution and the drospirenone solution to form a combined solution;
stirring the combined solution, preferably while keeping the solution at or below 25°C;
filtering the resultant precipitate;
washing the precipitate with a solvent selected from the group consisting of water, ethanol, ether and acetone, preferably wherein the solvent is cooled to below 25°C;
optionally suspending the resultant solid in a solvent, preferably acetone, and washing the suspended material with a solvent selected from the group consisting of water, ethanol, ether and acetone, preferably wherein the solvent Is cooled to below 25°C;
removing substantially all of the solvent from the resultant or suspended material.

9. The method according to claim 8, wherein the removing of solvent is by spray drying.

10. The method according to claim 8, wherein the removing of solvent is by lyophilization.

11. The method according to any of claims 3-10, further comprising combining a therapeutically active substance to the combined solution, the drospirenone solution, or the β-cyclodextrin solution.

12. A pharmaceutical composition comprising an inclusion complex of drospirenone and β-cyclodextrin as defined in claim 1 or 2 together with one or more pharmaceutically acceptable carriers or excipients.

13. The pharmaceutical composition according to claim 12 adapted to be administered by oral, parenteral, mucosal, or topical, vaginal, subcutaneous or nasal administration.

14. The pharmaceutical composition according to claim 12 or 13, wherein the amount of drospirenone is from 0.1 mg to 10 mg.

15. The pharmaceutical composition according to any of claims 12-14, further comprising one or more therapeutically active substances.

16. an inclusion complex as in claim 1 or 2 for use for female contraception.

17. An inclusion complex as defined in claim 1 or 2 for use in the treatment of menopausal symptoms.

## Patentansprüche

1. Einschlusskomplex von β-Cyclodextrin und Drospirenon, wobei der Einschlusskomplex von Drospirenon und dem β-Cyclodextrin in einem Molverhältnis von 1:3 vorliegt.

2. Einschlusskomplex nach Anspruch 1, weiterhin enthaltend eine oder mehrere therapeutische Wirkstoffe.

3. Verfahren zur Herstellung eines wie in Anspruch 1 definierten Einschlusskomplexes, bei dem man β-Cyclodextrin und Drospirenon in einem Molverhältnis von 0,3:1 bis 20:1 kombiniert.

4. Verfahren nach Anspruch 3, bei dem man eine Lösung von Drospirenon mit einer Lösung von β-Cyclodextrin kombiniert.

5. Verfahren nach Anspruch 3, bei dem man festes Drospirenon mit einer Lösung von β-Cyclodextrin kombiniert.

6. Verfahren nach Anspruch 4 oder 5, wobei die Lösung von β-Cyclodextrin ein aus der aus Wasser, Ethanol, Aceton, Acetonitril, Methanol, DMSO, Pyridin oder Kombinationen davon, vorzugsweise Wasser, bestehenden Gruppe ausgewähltes Lösungsmittel umfasst.

7. Verfahren nach Anspruch 4, wobei die Lösung von Drospirenon ein aus der aus Wasser, Ethanol, Aceton, Acetonitril, Methanol, DMSO, Pyridin oder Kombinationen davon, vorzugsweise Wasser oder Ethanol, bestehenden Gruppe ausgewähltes Lösungsmittel umfasst.

8. Verfahren nach Anspruch 3, welches die folgenden Schritte umfasst:
Lösen des β-Cyclodextrins in Wasser, gegebenenfalls unter Erhitzen, unter Bildung einer β-Cyclodextrinlösung;
Lösen des Drospirenons in einem aus der aus Wasser und Ethanol oder Mischungen davon bestehenden Gruppe ausgewählten Lösungsmittel, gegebenenfalls unter Erhitzen, unter Bildung einer Drospirenonlösung;
Kombinieren der β-Cyclodextrinlösung und der Drospirenonlösung unter Bildung einer kombinierten Lösung;
Rühren der kombinierten Lösung, wobei die Lösung vorzugsweise bei oder unter 25°C gehalten wird;
Abfiltrieren des erhaltenen Niederschlags;
Waschen des Niederschlags mit einem aus der aus Wasser, Ethanol, Ether und Aceton bestehenden Gruppe ausgewählten Lösungsmittel, wobei das Lösungsmittel vorzugsweise auf unter 25°C abgekühlt wird;
gegebenenfalls Suspendieren des erhaltenen Feststoffs in einem Lösungsmittel, vorzugsweise Aceton, und Waschen des suspendierten Materials mit einem aus der aus Wasser, Ethanol, Ether und Aceton bestehenden Gruppe ausgewählten Lösungsmittel, wobei das Lösungsmittel vorzugweise auf unter 25°C abgekühlt wird;
Entfernen von im Wesentlichen des gesamten Lösungsmittels aus dem erhaltenen bzw. suspendierten Material.

9. Verfahren nach Anspruch 8, wobei das Entfernen von Lösungsmittel durch Sprühtrocknen erfolgt.

10. Verfahren nach Anspruch 8, wobei das Entfernen von Lösungsmittel durch Lyophilisieren erfolgt.

11. Verfahren nach einem der Ansprüche 3-10, bei dem man weiterhin einen therapeutischen Wirkstoff mit der kombinierten Lösung, der Drospirenonlösung oder der β-Cyclodextrinlösung kombiniert.

12. Pharmazeutische 2usammensetzung, enthaltend einen wie in Anspruch 1 oder 2 definierten Einschlusskomplex von Drospirenon und β-Cyclodextrin zusammen mit einem oder mehreren pharmazeutisch unbedenklichen Trägern oder Hilfsstoffen.

13. Pharmazeutische 2zusammensetzung nach Anspruch 12, ausgelegt auf eine Verabreichung durch orale, parenterale, mukosale oder topische, vaginale, subkutane oder nasale Verabreichung.

14. Pharmazeutische 2zusammensetzung nach Anspruch 12 oder 13, wobei die Menge an Drospirenon 0,1 mg bis 10 mg beträgt.

15. Pharmazeutische 2zusammensetzung nach einem der Ansprüche 12-14, die weiterhin einen oder mehrere therapeutische Wirkstoffe enthält.

16. Einschlusskomplex nach Anspruch 1 oder 2 zur Verwendung bei der Empfängnisverhütung von Frauen.

17. Einschlusskomplex nach Anspruch 1 oder 2 zur Verwendung bei der Behandlung von menopausalen Symptomen.

## Revendications

1. Complexe d'inclusion entre de la β-cyclodextrine et de la drospirénone, où le complexe d'inclusion entre la drospirénone et la β-cyclodextrine est selon un rapport molaire de 1:3.

2. Complexe d'inclusion selon la revendication 1, comprenant en outre une ou plusieurs substances thérapeutiquement actives.

3. Méthode de production d'un complexe d'inclusion tel que défini selon la revendication 1, comprenant la combinaison de β-cyclodextrine et de drospirénone selon un rapport molaire allant de 0,3:1 à 20:1.

4. Méthode selon la revendication 3, comprenant la combinaison d'une solution de drospirénone avec une solution de β-cyclodextrine.

5. Méthode selon la revendication 3, comprenant la combinaison de drospirénone solide avec une solution de β-cyclodextrine.

6. Méthode selon la revendication 4 ou 5, dans laquelle la solution de β-cyclodextrine comprend un solvant choisi dans le groupe constitué par l'eau, l'éthanol, l'acétone, l'acétonitrile, le méthanol, le DMSO, la pyridine ou des associations de ceux-ci, préférablement l'eau.

7. Méthode selon la revendication 4, dans laquelle la solution de drospirénone comprend un solvant choisi dans le groupe constitué par l'eau, l'éthanol, l'acétone, l'acétonitrile, le méthanol, le DMSO, la pyridine ou des associations de ceux-ci, préférablement l'eau ou l'éthanol.

8. Méthode selon la revendication 3, comprenant les étapes consistant à :
solubiliser de la β-cyclodextrine dans de l'eau, éventuellement sous chauffage, afin de former une solution de β-cyclodextrine ;
solubiliser de la drospirénone dans un solvant choisi dans le groupe constitué par l'eau ou l'éthanol, ou des mélanges de ceux-ci, éventuellement sous chauffage, afin de former une solution de drospirénone ;
combiner la solution de β-cyclodextrine et la solution de drospirénone afin de former une solution combinée ;
agiter la solution combinée, préférablement tout en maintenant la solution à 25°C ou moins ;
filtrer le précipité résultant ;
laver le précipité par un solvant choisi dans le groupe constitué par l'eau, l'éthanol, l'éther et l'acétone, préférablement où le solvant est refroidi jusqu'à une température inférieure à 25°C ;
éventuellement mettre en suspension le solide résultant dans un solvant, préférablement l'acétone, et laver le matériau en suspension par un solvant choisi dans le groupe constitué par l'eau, l'éthanol, l'éther et l'acétone, préférablement où le solvant est refroidi jusqu'à une température inférieure à 25°C ;
éliminer sensiblement la totalité du solvant à partir du matériau résultant ou mis en suspension.

9. Méthode selon la revendication 8, dans lequel l'élimination du solvant se fait par séchage par pulvérisation.

10. Méthode selon la revendication 8, dans lequel l'élimination du solvant se fait par lyophilisation.

11. Méthode selon l'une quelconque des revendications 3-10, comprenant en outre la combinaison d'une substance thérapeutiquement active à la solution combinée, la solution de drospirénone, ou la solution de β-cyclodextrine.

12. Composition pharmaceutique comprenant un complexe d'inclusion de drospirénone et de β-cyclodextrine tel que défini selon la revendication 1 ou 2, conjointement avec un ou plusieurs véhicules ou excipients pharmaceutiquement acceptables.

13. Composition pharmaceutique selon la revendication 12, adaptée pour être administrée par administration orale, parentérale, mucosale, topique, vaginale, sous-cutanée ou nasale.

14. Composition pharmaceutique selon la revendication 12 ou 13, dans laquelle la quantité de drospirénone va de 0,1 mg à 10 mg.

15. Composition pharmaceutique selon l'une quelconque des revendications 12-14, comprenant en outre une ou plusieurs substances thérapeutiquement actives.

16. Complexe d'inclusion selon la revendication 1 ou 2, pour une utilisation destinée à la contraception féminine.

17. Complexe d'inclusion tel que défini selon la revendication 1 ou 2, pour une utilisation destinée au traitement des symptômes de la ménopause.
